Europäisches Patentamt

European Patent Office

Office européen des brevets

(19)

(11) **EP 0 740 800 B1**

(12) **EUROPÄISCHE PATENTSCHRIFT**

(45) Veröffentlichungstag und Bekanntmachung des Hinweises auf die Patenterteilung:
**28.04.2004 Patentblatt 2004/18**

(51) Int Cl.⁷: $G01S\ 13/56$, G01S 7/41

(86) Internationale Anmeldenummer:
**PCT/DE1995/000065**

(21) Anmeldenummer: **95906879.2**

(22) Anmeldetag: **20.01.1995**

(87) Internationale Veröffentlichungsnummer:
**WO 1995/020171 (27.07.1995 Gazette 1995/32)**

(54) **VERFAHREN ZUR ERFASSUNG VON VITALFUNKTIONEN LEBENDER KÖRPER**

PROCESS FOR DETECTING VITAL FUNCTIONS IN LIVING BODIES

PROCEDE POUR LA DETECTION DES FONCTIONS VITALES DES CORPS VIVANTS

(84) Benannte Vertragsstaaten:
**AT CH DE FR GB IE IT LI**

(30) Priorität: **20.01.1994 DE 9400950 U**

(43) Veröffentlichungstag der Anmeldung:
**06.11.1996 Patentblatt 1996/45**

(73) Patentinhaber: **SELECTRONIC Gesellschaft für Sicherheitstechnik und Sonderelektronik mbH 14542 Werder/Havel (DE)**

(72) Erfinder: **SCHMIDT, Gerd, Juergen D-60318 Frankfurt am Main (DE)**

(74) Vertreter: **Blumbach, Kramer & Partner GbR Patentanwälte, Alexandrastrasse 5 65187 Wiesbaden (DE)**

(56) Entgegenhaltungen:
FR-A- 2 645 307       US-A- 3 796 208
US-A- 3 815 131       US-A- 4 958 638

• **1979 CARNAHAN CONFERENCE ON CRIME COUNTERMEASURES, 16.Mai 1979 - 18.Mai 1979 LEXINGTON, KENTUCKY, Seiten 53-56, Y. LIPKIN ET AL. 'MICROWAVE RESPIRATION MONITOR (MWRM)'**

**Beschreibung**

Die Erfindung betrifft ein Verfahren nach Anspruch 1 sowie Verwendungen des Verfahrens.

Das Dokument US-A-4958638 betrifft eine Vorrichtung und ein Verfahren zur Erfassung lebender Körper mittels Abstrahlung elektromagnetischer Signale und Gewinnung von für lebende Körper charakteristischen spektralen Anteilen der reflektierten Signale. Hierbei wird eine synchrone Quadratur-Detektion eingesetzt, wobei ein zur Trägerfrequenz synchron laufendes Erfassungsverfahren verwendet wird.

Das Dokument "Proceedings of the '79 Carnahan Conference on Crime Countermeasures, Seiten 53-56, betrifft offenbart zur Erfassung lebender Körper den Einsatz eines Interferometers, in dem ein "macig T" dazu dient, dass das empfangene Signal mit dem abgestrahlten Signal in einem Diodendetektor gemischt wird.

Das Dokument US-A-3796208 beschreibt eine Erfassung lebender Körper mit einer Schaltungsanordnung, bei welcher mittels eines Trimmers eine Einstellung der Überlagerung zwischen empfangenen und gesendeten Signal vorgenommen wird.

Aufgabe der Erfindung ist es ein weiteres Verfahren zur Erfassung von Vitalfunktion lebender Körper anzugeben.

Die erfindungsgemäße Lösung der Aufgabe ist durch ein Verfahren nach Anspruch 1 gegeben.

Die Erfinder haben festgestellt, daß lebende Körper, somit auch lebende menschliche Körper, durch ihre wichtigsten Vitalfunktionen, d.h. durch ihren Herzschlag sowie durch ihre Atemtätigkeit hochfrequente elektromagnetische Signale in überraschender Weise beeinflussen können.

Da diese Vitalfunktionen in der Regel innerhalb bekannter Frequenzbereiche ablaufen, die bei der menschlichen Herzfrequenz von etwa 0,5 bis 3,4 Hz liegen können und normalerweise bei etwa 1 bis 2 Hz liegen, bei Atmung von 0,1 bis 1,5 Hz reichen, werden hierdurch charakteristische Frequenzintervalle definiert. Diese Frequenzintervalle werden beim Empfang und der Aufzeichnung von elektromagnetischen Signale dann sichtbar, wenn sich Personen im Empfangsbereich befinden. Darüberhinaus ist es möglich, anhand empfangener und auch verarbeiteter Signale eine Aussage über die Anzahl der georteten Personen zu treffen. Hierbei wird das Prinzip der biologischen Vielfalt sowie Spezifität genutzt, auf Grund dessen sich die Herz- und Atemfrequenzmuster verschiedener Personen unterscheiden. Ab einer Personenzahl von vier kann jedoch durch eine Frequenzüberlagerung der jeweiligen Frequenzen im allgemeinen nicht mehr eindeutig unterschieden werden. Ab dieser Personenzahl ist dann nur noch die Aussage möglich: 'Es sind mindestens vier Personen anwesend'.

In jedem Falle umfaßt ein Frequenzbereich von 0,01 bis 10 Hz alle für die Vitalfunktionen eines menschlichen Körpers interessierenden Frequenzen.

Überraschend war die Erkenntnis, daß auch ohne abgestrahlte Sendeleistung lediglich eine Empfangseinrichtung zusammen mit der Einrichtung zur Gewinnung der für lebende Körper charakteristischen Frequenzanteile in der Lage war, den erwünschten Nachweis der Vitalfunktionen zu erbringen.

Dies bedeutet, daß bereits das Vorhandensein eines lebenden Körpers zumindest in der Nähe der Empfangseinrichtung zu nachweisbaren Signalanteilen in den erwähnten Frequenzbereichen führt, ohne daß dabei eine Durchstrahlung mit einem Trägersignal vorgenommen werden mußte.

Die Erfinder waren bereits mit der Empfangseinrichtung für elektromagnetische Signale und der Einrichtung zur Gewinnung von für lebende Körper charakteristischen Frequenzanteilen ohne zusätzliche abgestrahlte Signale in der Lage lebende Körper bis zu mehr als 3 m Entfernung oder in etwa der Entfernung eines Gebäudestockwerks sicher zu erfassen.

In der einfachsten Ausführungsform der Erfindung reichte bereits die Verwendung des später beschriebenen Direktdemodulators in Form eines Diodendirektempfängers für den Empfang der für lebende Körper charakteristischen Frequenzanteile aus.

Man setzte später zusätzlich Sender ein, mit welchen eine Durchstrahlung des Erfassungsgebietes vorgenommen wurde, und empfing reflektierte, transmittierte oder gestreute Strahlung, deren Untersuchung auf vorstehende Frequenzanteile den Nachweis für das Vorhandensein der zu überwachenden Vitalfunktionen lieferte.

Um elektromagnetische Strahlung für Überwachungs- oder Sicherungszwecke noch in einiger Entfernung empfangen zu können, wurden Frequenzen der elektromagnetischen Strahlung von einigen hundert Megahertz bis etwa 10 Gigahertz eingesetzt, die eine hohe Eindringtiefe sicherstellten.

Diese Strahlung erfuhr eine Phasenmodulation, die dem hochfrequenten Trägersignal um einige Hertz verschobene Seitenbänder zufügte. Ein Nachweis derart nahe beieinander liegender Frequenzbänder hätte mit herkömmlichen Empfangstechniken kurzzeitstabile Oszillatoren mit Abweichungen von weniger als $10^{-12}$ erfordert, was bisher bei vertretbarem Aufwand als unerreichbar galt. Dieses Problem wird durch die in der Regel geringen empfangenen Signalleistungen weiter verschärft.

Zunächst erschien zum Empfangen der elektromagnetischen Signale die Verwendung bekannter Phasendemolulatoren als naheliegend. Bekannt sind Homodyn-, Heterodyn-, PLL- (Phase Locked Loop-, phasenverrastete Schleifen-) Verfahren sowie die Anregung der Flanke eines lokalen Schwingkreises. Es hat sich jedoch herausgestellt, daß keines der vorstehenden Verfahren in der Lage war, mit vertretbarem Aufwand die erwünschten Ergebnisse zu liefern. Erst der Einsatz eines Direkt-Demodulators, mit welchem eine direkte Trennung der Modulationsfrequenz von der modulierten Frequenz ermöglicht wird, führte zu den gewünschten Ergebnissen. Es wird jedoch davon ausgegangen, daß bei entsprechendem apparativem Aufwand sowie verbesserten Schaltungsanordnungen vorstehende Empfangs-Verfahren im Rahmen der Erfindung anwendbar werden können.

Mit einem Bauelement mit nichtlinearer Strom/Spannungskennlinie als frequenzselektivem Element konnte die Demodulation der interessierenden Frequenzanteile kostengünstig und zuverlässig erreicht werden. Als Element mit nichtlinearer Kennlinie konnte eine Diode, ein bipolarer oder ein Feldeffekttransistör mit Erfolg eingesetzt.

Das Dokument "Schottky Barrier Diode Video Detectors; Application Note 923", Hewlett-Packard 1986, zeigt wie eine Diode zur Direktdemodulation eines Videosignals verwendet werden kann.

Diese Bauteile sind sowohl preisgünstig erhältlich als auch unkritisch bei ihrer Verwendung. Der optimale Arbeitsbereich dieser Bauteile von etwa 100 kHz bis 200 MHz konnte bei höheren Empfangsfrequenzen durch eine dem Demodulator vorgeschaltete Frequenzumsetzeinrichtung genutzt werden. Diese Frequenzumsetzeinrichtung fügte dem Signal zwar tolerierbare Verzerrungen im Zeitbereich hinzu, überlagerte jedoch nur geringes zusätzliches Rauschen.

Mit einer Sendeeinrichtung zum Senden eines elektromagnetischen Trägersignals mit festgelegter Frequenz konnte das zu empfangende Signal angehoben werden; höchste Aufmerksamkeit mußte jedoch der Stabilität der Trägerfrequenz gewidmet werden, um unerwünschte Modulationen im interessierenden Frequenzbereich auszuschließen. Eine einfache quarzstabilisierte analoge Sendeschaltung mit einem Schwingkreis hoher Güte zeigte sich überaschenderweise nach ausreichender Einschwingzeit als geeigneter Oszillator.

Das erfindungsgemäße Verfahren ist ebenfalls zur Objektüberwachung und/oder - sicherung verwendbar.

Der Einsatz eines analogen Abtastfilters zeigte anders als hochfrequente digitale Filtern keinerlei abträgliche zusätzliche Frequenzkomponenten und trug maßgeblich mit zur Qualität des erhaltenen Signals bei. Zusätzliche unerwünschte Signalanteile, wie z.B. Rauschen und überlagerte Störungen, wurden durch Begrenzung der Bandbreite des elektromagnetischen Signals vor der Abtastung und vor der A/D-Wandlung zu hohen Frequenzen hin vermieden.

Wichtig war auch der Einsatz eines analogen Hochpaßfilters zum Vermeiden niederfrequenter Anteile des frequenzabhängigen 1/f-Rauschens des Sendeoszillators und interner Baugruppen.

Das unerwartet gute Funktionieren des erfindungsgemäßen Verfahrens läßt deren Verwendung auf vielen Gebieten zu.

Im Justizvollzug oder der Psychiatrie können suizidgefährdete Personen überwacht werden, ohne daß es der ständigen Aufsicht durch Betreuungspersonal bedarf.

Die Erfindung wird nachfolgend unter Bezugnahme auf die beigefügten Zeichnungen anhand beispielhafter Ausführungsformen, die zur Durchführung des erfindungsgemäßen Verfahrens benutzt werden können, im einzelnen beschrieben.

Es zeigen:

Fig. 1      eine schematische Darstellung der Hauptbaugruppen einer Vorrichtung, die zur Durchführung der Erfindung verwendet werden kann.

Fig. 2      eine schematische Darstellung einer einfacheren Einrichtung mit deren Hauptbestandteilen,

Fig. 3      eine schematische Darstellung des Aufbaus der Auswertekette,

Fig. 4 und 4a      ein Flußdiagramm der implementierten Bearbeitungsschritte,

Fig. 5 und 6      spektrale Darstellungen von mit der Vorrichtung erfaßten elektromagnetischen Signalen mit für die Vitalfunktionen lebender menschlicher Körper charakteristischen Frequenzanteilen,

Fig. 7      einen Diodendirektempfänger ohne vorgeschalteten Konverter,

Fig. 8a      ein Schaltbild eines analogen Hochpaßfilters und eines als Tiefpaß ausgebildeten Anti-Aliasing-Filters,

Fig. 8b      ein Schaltbild der Spannungssymmetrisierung,

Fig. 9      eine erste Ausführungsform zur Überwachung von Vitalfunktionen, von der Seite her gesehen,

Fig. 10      eine Ansicht des Kopfes der in Fig. 9 dargestellten Ausführungsform von unten,

Fig. 11      eine zweite Ausführungsform zur ortsfesten Montage, von der Seite her gesehen,

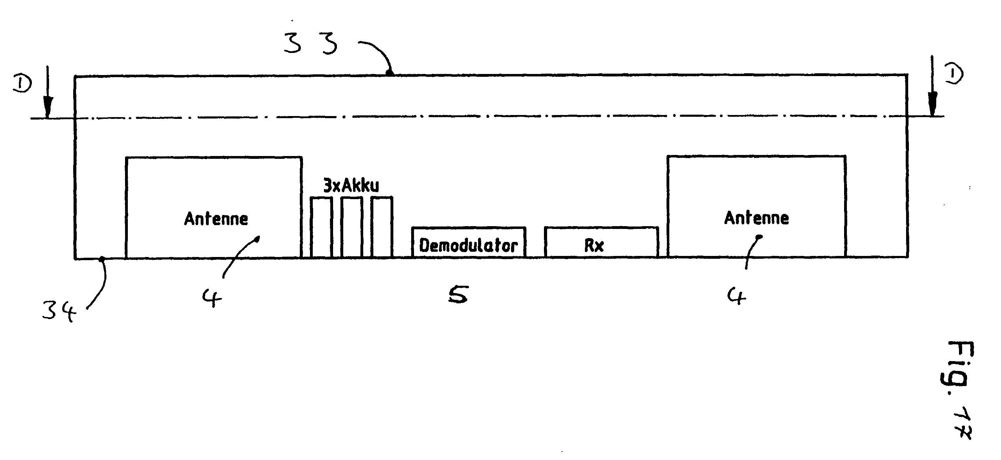

Fig. 12      eine Schnittdarstellung entlang der Linie A-A aus Fig. 11 durch die zweite Ausführungsform,

Fig. 13      eine dritte Ausführungsform zur Raumüberwachung,

Fig. 14      eine alternative der in Fig. 13 dargestellten Anordnung zur Überwachung mehrere Gebäudegeschosse,

Fig. 15      eine weitere Anordnung zur Raum- und/oder Gebäudeüberwachung,

Fig. 16      eine nochmals weitere Anordnung zur Raum- sowie Vorfeldüberwachung von Gebäuden.

Nachfolgend wird anhand einzelner Ausführungsformen beschrieben, wie das erfindungsgemäße Verfahren durch geführt werden kann.

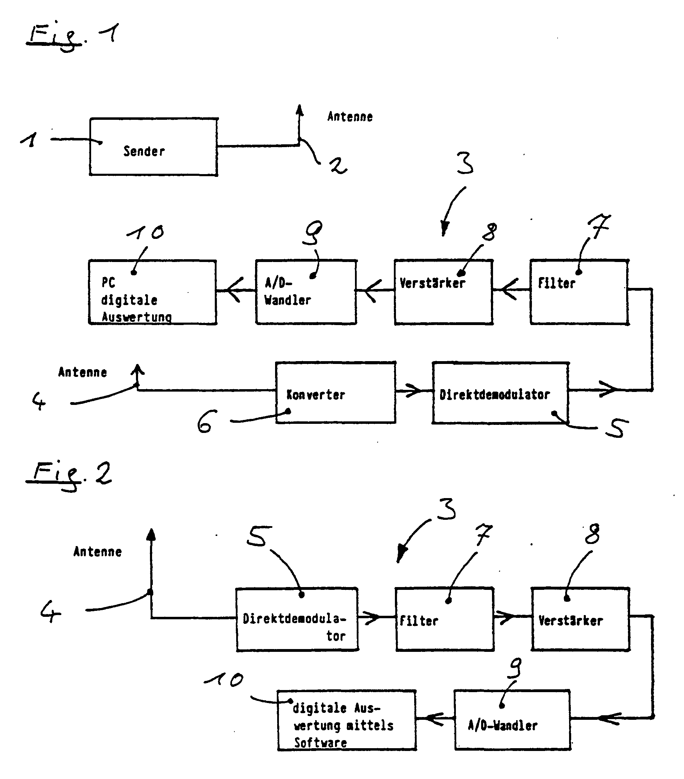

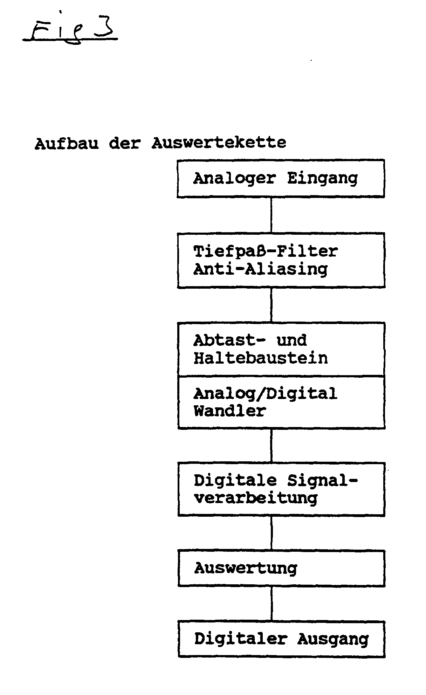

Fig. 1 zeigt eine Anordnung mit einem Sender 1 und einer Sendeantenne 2, die auf einer festen Frequenz senden, welche vorzugsweise im Bereich einiger 100 MHz bis etwa 10 GHz liegt.

Die Sendeantenne 2 hat vorzugsweise eine keulenförmige, festgelegte Richtcharakteristik. Der Sender 1 und die Sendeantenne 2 sind je nach Ausführungsform als portable Einheit ausgebildet oder stationär montiert.

Die im Ganzen mit 3 bezeichnete Empfangseinrichtung, die in einer einfacheren Ausführungsform in Fig. 2 dargestellt ist, umfaßt eine Empfangsantenne 4, die mit einem Direktmodulator 5 verbunden ist, der aus dem empfangenen elektromagnetischen Signal die für lebende Körper charakteristischen Frequenzanteile demoduliert. Diese Demodulation wird als Phasen- oder Frequenzdemodulation durchgeführt und kann bereits am Ausgang des Direktdemodulators 5 die erwünschten Frequenzanteile bereitstellen.

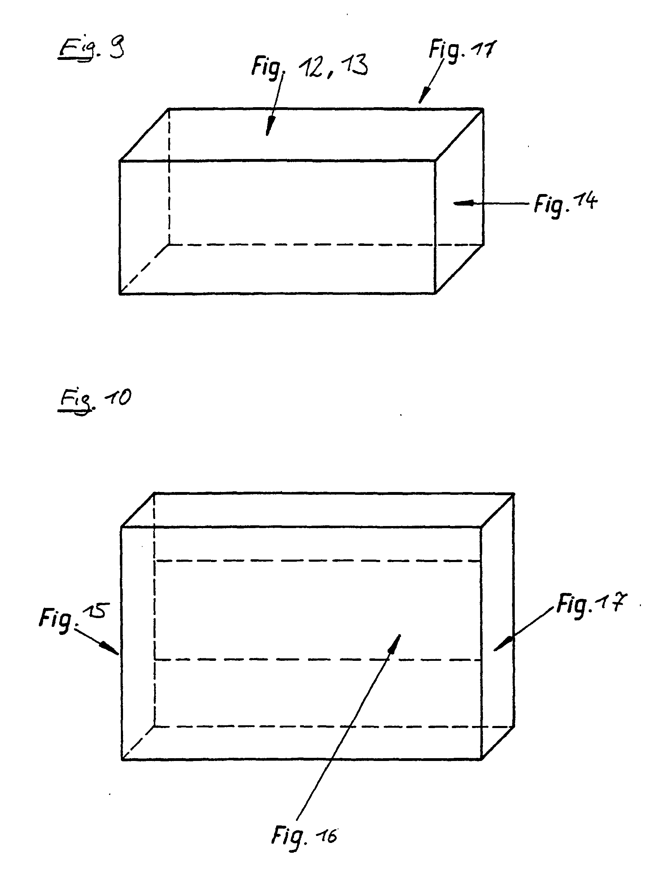

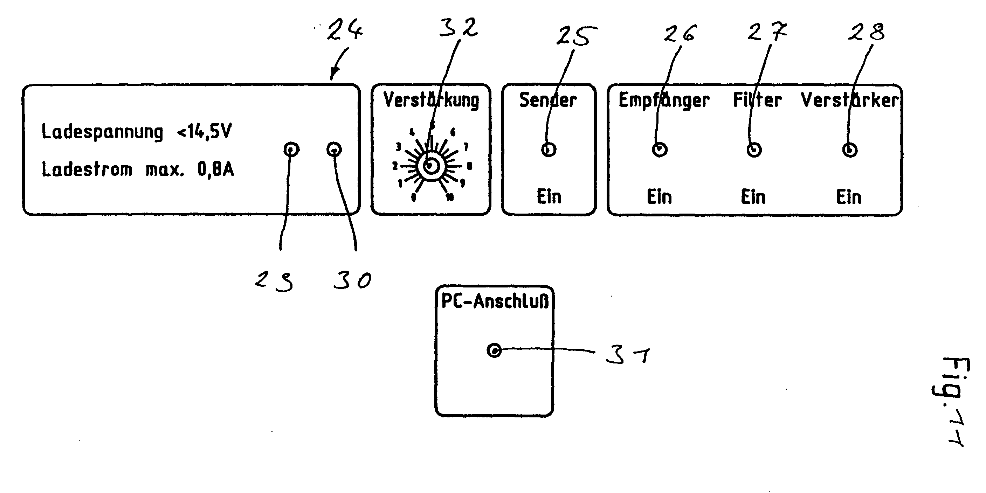

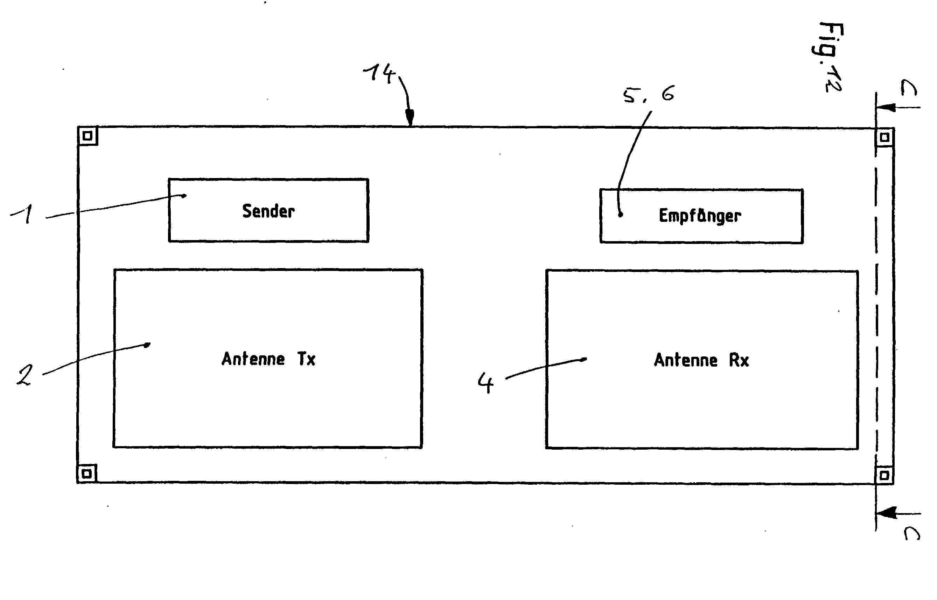

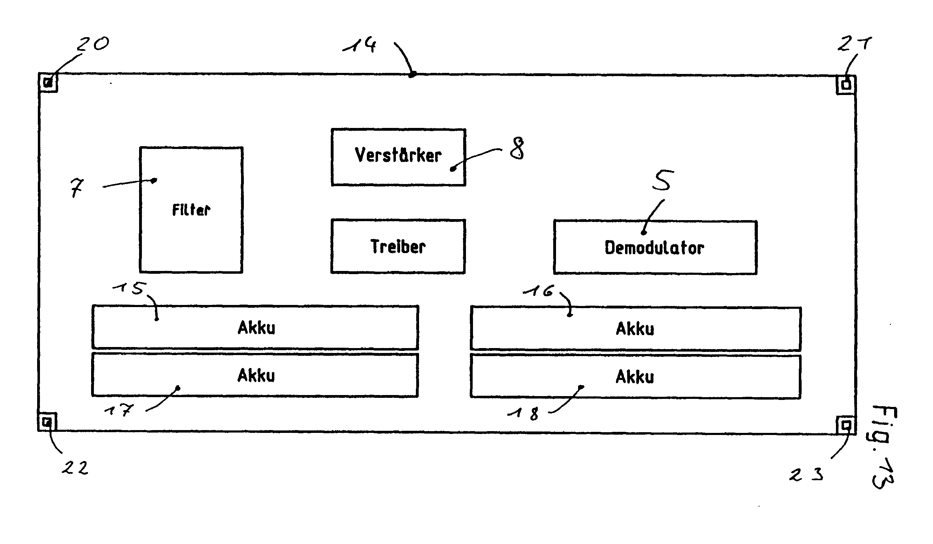

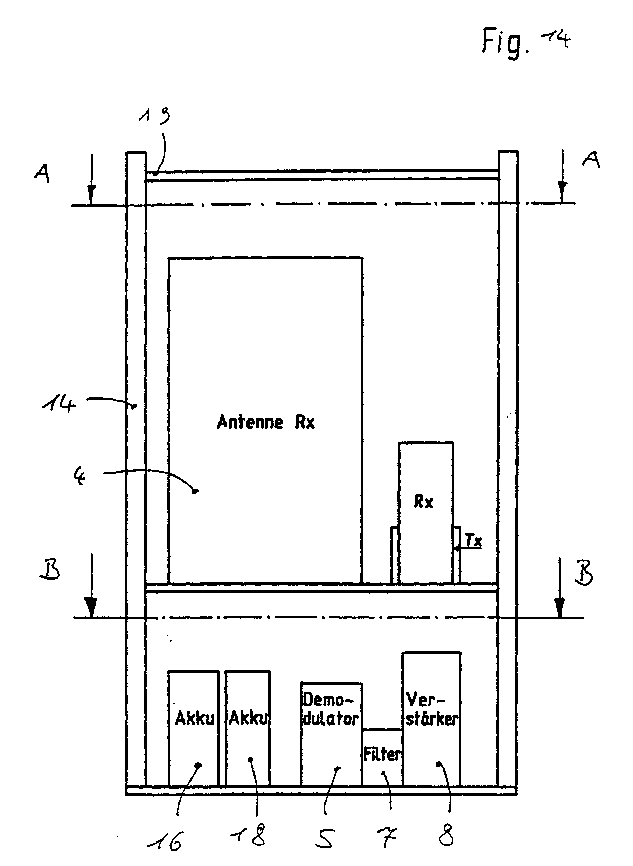

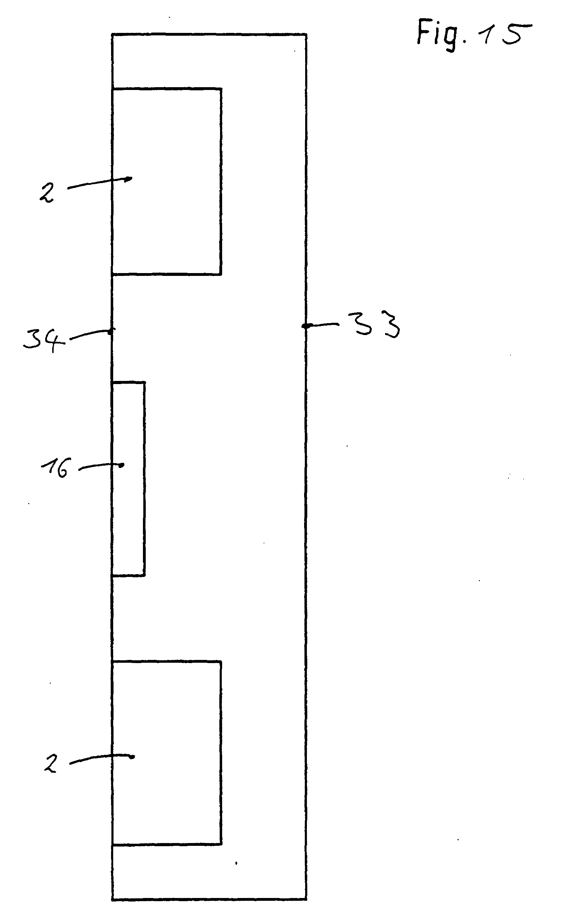

Gegenüber der in Fig. 7 dargestellten Ausführungsform des Direktdemodulators kann dieser auch aus einer Gleichrichterbrücke bekannter Bauart bestehen, die zu einem spannungsverdoppelten oder spannungsvervierfachten Nutzsignal führt.

In weiterer Ausgestaltung umfaßt die Empfangseinrichtung 3 eine dem Demodulator 5 vorgeschaltete Frequenzumsetzeinrichtung 6, die als Konverter oberhalb von ca. 200 Megahertz bis Terrahertz empfangende Signale in Frequenzbereiche umsetzt, bei welchen der Direktdemodulator 5 erhöhte Empfangsleistungen zeigt. Bei Verwendung von Dioden, einem bipolaren oder einem Feldeffekttransistor liegt dieser geeignete, abwärts konvertierte, optimale Arbeitsbereich bei etwa 100 kHz bis 200 MHz.

Dem Direktdemodulator nachgeschaltet ist eine Filtereinrichtung 7 zum Ausfiltern unerwünschter Signalanteile, welche die Bandbreite des elektromagnetischen Signals vor der Abtastung (vor der Analog/Digital-Wandlung) zu hohen Frequenzen hin begrenzt. Diese Filtereinrichtung 7 begrenzt die Bandbreite ebenfalls zu niedrigen Frequenzen hin. Der dem Filter 7 nachgeschaltete Verstärker 8 erhöht die Spannung oder in alternativer Ausgestaltung den Strom der empfangenen Signale und führt diese zur Abtastung einem Analog/Digital-Wandler 9 zu.

Nach Analog/Digital-Wandlung werden die für lebende Körper charakteristischen Frequenzanteile durch eine Recheneinrichtung 10 zur Spektralanalyse aufbereitet und spektral dargestellt. Hierbei gibt die Intensität der Frequenzanteile, die für lebende Körper charakteristisch sind, Aufschluß über das Vorhandensein der Vitalfunktionen der erfaßten menschlichen Körper.

Bei zeitlicher Auswertung der Signale wird das digitale Signal zu seiner Entzerrung mit der inversen Transferfunktion der Empfangseinrichtung 3 gefaltet.

Da der zuverlässige Nachweis dieser Signale äußerst schwierig ist, sei nachfolgend der Direktempfänger mit dem nichtlinearen Element anhand eines Diodendirektempfängers beschrieben.

Diodendirektempfänger

Das reflektierte Signal ist phasen- oder frequenzmoduliert. Der Nachweis dieser Modulation ist mit den üblichen Empfangstechniken für FM (Frequenzmodulation) und PM (Phasenmodulation) nicht oder nur unter extremen Schwierigkeiten möglich. Um ein mit 0,2 Hz phasenmoduliertes Signal z.B. bei 10 GHz auf $0,2 \pm 0,02$ Hz genau nachzuweisen, wären kurzzeitstabile, synchronisierte Oszillatoren mit Abweichungen von kleiner 10-12 notwendig. Dies erschien bisher als technisch nicht realisierbar.

Es wurde daher nach einem Weg gesucht, die Modulation des empfangenen Signals direkt nachzuweisen.

Hierfür eignen sich beispielsweise Bauelemente mit weitestgehend quadratischen Kennlinien; dies sind unter anderem Feldeffekt-Transistoren, Bauelemente mit exponentiellen Kennlinien, die stückweise als quadratisch approximiert werden können, Dioden und Transistoren. Wird nun als eingeprägte, empfangene Spannung die Summe zweier Frequenzen angelegt, so entstehen Terme höherer Ordnung.

Ist ein quadratischer Term vorhanden, so treten neben dem Richtstrom auch Differenzfrequenzen auf. Um das phasenmodulierte Signal, welches von der zu detektierenden Person reflektiert wird, zu demodulieren, kann somit in üheraschender Weise trotz höchster Anforderungen an das Frequenzverhalten bereits ein gewöhnlicher Gleichrichter eingesetzt werden.

An der nichtlinearen Kennlinie wird das phasenmodulierte Signal eingeprägt, und es entstehen Ströme, die proportional der Phasenmodulationsfrequenz $\Omega$ und deren Vielfachen $k*\Omega$ sind. Die Kurvenform der Modulation bleibt aufgrund des Demodulationsprinzips nicht erhalten, es hat sich jedoch herausgestellt, daß diese Änderungen der Kurvenform für die meisten erfindungsgemäßen Anwendungen unkritisch sind, da für diese der Nachweis der Modulation ausreichend sein kann.

Das Signal-Rausch-Verhältnis bestimmt bei direktem Nachweis die Empfindlichkeitsgrenze. Für die Atmungsfrequenz wurden S/N-Werte über 46 db, für den Herzschlag Werte von 26 dB in einer Entfernung von 3 m und bei Oszillatorleistungen von ca. 5 mW erzielt.

Mit der Annahme, daß vom Herzen Kugelwellen emittiert werden, besteht zwischen Sende- und Empfangsleistung eine zur zweiten Potenz der Entfernung umgekehrt proportionale Beziehung. Für das Verhältnis der Amplituden der Atmungsfrequenz UA zum Rauschen UN bzw. der Herzfrequenz UH zum Rauschen kann daher abgeschätzt werden, daß die Empfangsgrenze bei einer Sendeleistung von 1 W dann bezüglich des Herzschlags bei ca. 50 m und bezüglich der Atmung bei typ. 160 m liegt.

Antennen mit höherem Gewinn und rauscharme Komponenten können diese Werte in erfindungsgemäßer Weise entsprechend erhöhen.

Die bezüglich Sättigungsstrom I0 und Temperaturspannung ideale Diode ist die Si-Leistungsdiode 1N4004, deren Eignung als Gleichrichter allerdings zu hohen Frequenzen durch die große Sperrschichtkapazität eingeschränkt ist. Danach folgt die Kleinsignal Si-Diode 1N4148, dann die Si-Schottky-Diode BAT 46 und schließlich die beiden Ge-Dioden AA116 und AA144.

Ein Diodendirektempfänger wurde jeweils für 440 MHz, 1,3 GHz, 2,4 GHz, 5,6 GHz und 10 GHz abgeglichen. Für 4 der 5 Frequenzen wurden Empfangsantennen mit Diodendirektempfänger aufgebaut:

440 MHz : Halbwellendipol mit v=0,940, Z=60,5 Ω und BAT 46

1,3 GHz : Halbwellendipol mit v=0,906, Z=57,4 Ω und BAT 46

2,4 GHz : Halbwellendipol mit v=0,940, Z=60,5 Ω und BAT 46

5,6 GHz : Ganzwellen-Dreieckflächendipol mit v=0,73, Z=140 Ω und BAT 46

Bereits bei diesem Empfänger sank die Empfindlichkeit gegenüber dem 2,4 GHz Empfänger stark ab. Bei 10 GHz war keine verwertbare Spannung mehr nachweisbar, so daß auf den Bau eines 10 GHz Diodendirektempfängers verzichtet wurde. Die zur Verfügung stehenden Dioden zeigen bei derart hohen Frequenzen keinen verwertbaren Gleichrichtereffekt mehr.

Da erfindungsgemäße Signale von Experten als unterhalb der Meßgrenze liegend eingestuft worden waren, ist den verwendeten Antennentypen starke Aufmerksamkeit geschenkt worden.

Antennen

Das Vor-Rück-Verhältnis muß so groß wie nur möglich gemacht werden, um keine Signale zu empfangen, die entgegen der Hauptabstrahlrichtung einfallen. Auch Nebenkeulen müssen aus diesem Grunde minimiert werden. Das gesamte Strahlungsdiagramm sollte somit eine möglichst enge Hauptkeule und keine Nebenkeulen aufweisen.

Die Eingangsimpedanz der Antennen kann und soll in erfindungsgemäßer Weise so an reele oder komplexe Impedanzen angepaßt werden, daß bei Sendern eine Leistungsanpassung und bei Empfängern eine Rauschanpassung erzielt wird. Die Erfüllung dieser Forderungen durch eine Antennenbauform ist jedoch nicht gleichzeitig möglich.

Alle verwendeten Antennen sind Endfire-Antennen, da Backfire-Antennen vergleichbarer Abmessungen stets ein schlechteres Vor-Rück-Verhältnis aufweisen, da die Wellenleiterstruktur in Rückwärtsrichtung angeregt werden muß. Die Antennen sollten so breitbandig wie möglich sein, da auf einen Abgleich verzichtet werden sollte. Als breitbandige Antennen mit sehr gutem Vor-Rück-Verhältnis sind logarithmisch periodische Strukturen bekannt. Durch die logarithmische Stufung der Wellenleiterstrukturen wird einerseits Breitbandigkeit und andererseits eine ausgeprägte Richtwirkung erreicht. Daß der Gewinn verglichen mit resonanten Antennen vergleichbarer Abmessung geringer ausfällt, stört für den erfindungsgemäßen Anwendungsfall in der Regel nicht.

Die Polyconeantenne kann die Rotationsparaboloidantenne ersetzen, da Abweichungen von der Paraboloidgestalt, die kleiner als ein Zehntel Wellenlänge sind, sich nicht negativ auf das Verhalten der Antenne auswirken. Selbst bei einem Fünftel der Wellenlänge bleibt der Verlust an Verstärkung unter 2 dB und kann somit für die meisten Fälle vernachlässigt werden.

Die technisch aufwendig zu realisierende Bauform des Paraboloidreflektors kann somit ohne Nachteile durch den einfacher realisierbaren Polycone-Reflektor ersetzt werden. Die Speisung ist allerdings vergleichbar aufwendig, und auch das Vor-Rück-Verhältnis verbessert sich erst mit Reflektoren, die groß gegenüber der Wellenlänge sind und deren Ausleuchtung sich auf den inneren Bereich beschränkt.

Um den Problemen mit der Polarisation aus dem Weg zu gehen, wurden bei unseren Ausführungsformen mit den beiden höheren Frequenzen (5,6 GHz und 10,368 GHz) jeweils eine zirkular polarisierte Antenne - einmal als Empfangsantenne, einmal als Sendeantenne - verwendet. Dadurch treten zwar sicher Verluste von typisch 3 dB auf, diese sind aber klein im Vergleich zu den Verlusten, die bei gegeneinander verdrehten linear polarisierten Antennen entstehen können.

Mit einem Zirkulator konnte bei einer Ausführungsform mit nur einer gemeinsamen Sende/Empfangsantenne erfolgreich die ein- und auslaufenden Wellen getrennt werden.

Aufgrund der zu bewältigenden schwierigen meßtechnischen Verhältnisse wurde auch den Hochfrequenz-Baugruppen besondere Aufmerksamkeit geschenkt.

Hochfrequenz-Baugruppen

Die benötigten Hochfrequenz-Baugruppen sind nachfolgend aufgeführt. Die Aufstellung berücksichtigt die möglichen Verknüpfungen, die zwischen den Modulen und den peripheren Elementen bestehen. Diese entsprechen den von uns realisierten Konfigurationen.

Die Direktmodulatoren werden bei den höheren Frequenzen, d.h. bei Frequenzen oberhalb von ca. 200 MHz nach den auf die Zwischenfrequenz von 137,5 MHz umsetzenden Konvertern eingesetzt. Bei dieser Frequenz sind sowohl die verwendeten Dioden als auch die Transistoren funktionsfähig.

## 1. Diodenmischer

Der Diodenmischer besteht aus einer symmetrischen Spannungsvervierfacherschaltung mit einem Resonanzkreis am Eingang und einem Tiefpaß am Ausgang.

Im Gegensatz zu der bei der Diode als Direktempfänger erzielbaren Spannung kann hier die vierfache Ausgangsspannung erzielt werden, da die Quellen nun in Serie geschaltet sind. Der dadurch bedingte erhöhte Innenwiderstand ist für die Funktion unerheblich.

Im praktischen Betrieb zeigte sich, daß der Diodenmischer bezüglich des Signal-Rausch-Verhältnisses den anderen bekannten Mischerbauformen überlegen ist.

## Niederfrequenz-Baugruppen

Sämtliche im Niederfrequenzbereich betriebenen Module sind mit einer eigenen Stromversorgung ausgerüstet. Dazu wurden einzelne Bleiakkus mit 12 V/2Ah verwendet, die mit einer Spannungsüberwachungsschaltung und einem Ein-Schalter versehen wurden. Die strikte Trennung aller Energieversorgungseinheiten stellte sich als notwendig heraus, nachdem bereits die Verwendung eines Netzteils zu erheblichen Störungen führte.

Die gesamte Anordnung ist somit auf der Senderseite vollständig isoliert und auf der Empfängerseite nur über den Personal Computer mit dem Netz verbunden, der aber bei portablen Vorrichtungen als batteriebetriebenes Gerät ausgebildet ist.

## 1. Vorverstärker

Der Vorverstärker verwendet einen rauscharmen Vierfach-Operationsverstärker. Einer der Verstärker ist als Betriebsspannungssymmetrierer geschaltet; die anderen drei sind als Bandpaßfilter beschaltet und über Hochpaßfilter miteinander gekoppelt.

Ein Tiefpaß begrenzt das Rauschen der ersten Stufe. Durch einen optionalen Widerstand konnte der Diodendirektempfänger vom Vorverstärker aus mit einem Vorstrom versorgt werden. Insgesamt wurden zwei Vorverstärker-Module mit unterschiedlicher Verstärkung eingesetzt. Da die Empfindlichkeit der gesamten Anordnung zu einer Übersteuerung des A/D-Wandlers und damit zu einem Datenverlust führen kann, ist ein geregelter Verstärker notwendig.

## 2. Abtastfilter (Anti-Aliasing-Filter)

Die Abtastung zeitabhängiger Signale muß mit einer Frequenz erfolgen, die größer als doppelt so hoch ist wie die höchste, im Eingangssignal enthaltene Frequenz. Daher muß das Eingangssignal vor der Analog-Digital-Wandlung spektral begrenzt werden. Diese Begrenzung muß erstaunlicherweise für die Zwecke vorliegender Erfindung durch ein analoges Filter erfolgen und kann nicht durch digitale Verarbeitung ersetzt werden. Wird dies nicht berücksichtigt, so erfolgt eine Unterabtastung der spektralen Anteile, die sich oberhalb der halben Abtastfreqeunz befinden. Diese werden in den unteren Frequenzbereich gemischt und verfälschen das Signal irreversibel und es kann somit der erfindungsgemäße Erfolg nicht erreicht werden.

Sogenannte digitale Anti-Aliasing-Filter, die den Anwender glauben lassen, die Bandbegrenzung nach dem A/D-Wandler vornehmen zu können, erwiesen sich überaschend als völlig wirkungslos bezüglich des Problems; alle mit der Unterabtastung verknüpften Fehler traten auf. Eine nachträgliche digitale Korrektur war aufgrund des zerstörten Signalinhaltes nicht mehr möglich.

Generell ist festzuhalten, daß unter Fachleuten bezüglich der analogen und digitalen Kenngrößen derart falsche Vorstellungen vorherrschen, daß die Auslegung eines Meßsystems zur digitalen Verarbeitung analoger Größen aufgrund der Angaben der Hersteller und der ausschließlichen Verwendung der von diesen angebotenen Hard- und Software nicht zum Ziel führen konnte.

Die Anforderungen, die an das analoge Anti-Aliasing-Tiefpaßfilter gestellt werden, sind je nach Weiterverarbeitung sehr hoch. So muß der Dynamikbereich mindestens 1 Bit besser sein als der des nachfolgenden A/D-Wandlers und ebenso müssen lineare und nichtlineare Verzerrungen mindestens 1 Bit besser sein als der A/D-Wandler. Obwohl der Dynamikbereich eines N-Bit A/D-Wandlers in der Praxis meist nur N-2 Bits beträgt, müssen diese Zusammenhänge berücksichtigt werden. Die Verwendung von Schalter-Kondensator-Filtern ist möglich, falls auch dort das Abasttheorem berücksichtigt wird und der erzielte Dynamikbereich ausreichend ist.

Die Faltung des Eingangssignals mit dem Abtastfilter führt zu Amplituden- und Phasenverzerrungen und aufgrund der Gruppenlaufzeit des Filters zu Hüllkurvenverzerrungen. Diese Signalveränderungen können bei Bedarf berücksichtigt werden, indem die inverse Transferfunktion des Abtastfilters mit dem abgetasteten Signal im Rechner gefaltet wird. Diese Prozedur ist nur dann möglich, wenn korrekt abgetastet wurde. Im Falle einer Unterabtastung wird dagegen der Fehler weiter vergrößert.

Zwischen oberer Signalfrequenz fs, Abtastfrequenz fa, asymptotischer Steilheit bzw. Ordnung des Abtastfilters N und dem Überabtastungsfaktor k besteht folgender Zusammenhang zur erzielbaren Genauigkeit bzw. Auflösung A in Bit:

$$k = \frac{\ln(fa) - \ln(fs)}{\ln(2)} - 1$$

$$A = k*N + 1$$

Für eine Grenzfrequenz von fs = 2 Hz bei einer Auflösung von A = 13 Bit ergeben sich z.B. folgende Möglichkeiten zur Realisierung:

> Filter 1.ter Ordnung (N=1) ==> Abtastfrequenz fa=16384 Hz
> Filter 3.ter Ordnung (N=3) ==> Abtastfrequenz fa=64 Hz
> Filter 6.ter Ordnung (N=6) ==> Abtastfrequenz fa=16 Hz

Die letzte Kombination ist die in unseren Ausführungsfomen verwendete Anordnung. Bei Filtern geringer Ordnung mit "gutmütigem" Verhalten bezüglich der Transferfunktiön muß überaschender Weise mit extremen Überabtastraten gerechnet werden, um brauchbare Ergebnisse zu erzielen. Trotz der hohen Abtastfrequenz von über 16 kHz werden nur die Spektralanteile bis 2 Hz korrekt abgetastet. (Bei A=16 Bit, fs=20 kHz und fa=44 kHz wären Filter 109.ter Ordnung notwendig, um eine Abtastung entsprechend dem Abtasttheorem durchzuführen.)

Die Überabtastung besitzt einen weiteren Vorteil: Jeder Analog-Digital-Wandler, auch wenn er ideal bezüglich seiner Kennlinie ist, fügt dem abzutastenden Signal das Quantisierungsrauschen hinzu, so daß das Signal nicht nur durch die Quantisierung, d.h. die Diskretisierung der Amplitudenwerte, verfälscht wird, es wird zudem noch zusätzlich verrauscht.

Das Rauschen kann näherungsweise als weiß angesehen werden, so daß bei größerer Abtastbandbreite, d.h. bei Überabtastung, in die Signalbandbreite entsprechend weniger Rauschen fällt und somit das Signal-Rausch-Verhältnis des Wandlers, nicht aber des Signals, proportional verbessert werden kann.

Der verwendete Abtast-Tiefpaß 6.ter Ordnung wird durch die Reihenschaltung zweier Tiefpässe 3.ter Ordnung (asymptotische Flankensteilheit 18 dB/Oktave bzw. 60 dB pro Dekade) realisiert. Jeder Tiefpaß besteht aus einem als Spannungsfolger geschalteten Operationsverstärker und einer R-C-Beschaltung.

Die Amplituden, Phasen- und Hüllkurvenverzerrungen durch den Frequenz- und Phasengang aller Filter sowie die Gruppenlaufzeiten können rückgängig gemacht werden, indem die Zeitfunktion mit deren inversen Transferfunktionen T-1(w) des vorangegangenen Signalpfades gefaltet wird und somit eine vollständige Pol-Nullstellen-Kompensation durchgeführt wird. Dies kann notwendig werden, wenn das originale Zeitsignal rekonstruiert werden soll und daher die Verformung des Zeitsignals durch die Wandler und die Elemente der Übertragungskette vermieden werden muß. In einem Anwendungsfall, in welchem der signifikante Nachweis einer Spektrallinie erforderlich ist, kann davon abgesehen werden.

In dem Verwendeten Aufbau durchläuft bei einer Ausführungsform das Zeitsignal vom Wandler (Empfangsantenne) bis zum Personal Computer (A/ D-Wandler) mindestens einen Hochpaß 15.ter Ordnung und einen Tiefpaß 21.ter Ordnung, die sich aus dem Produkt der Transferfunktionen der einzelnen Elemente der Meßkette ergeben (Direktmischer, Vorverstärker, 2*Tiefpaß, 2*Hochpaß, A/D-Wandler).

Das dynamische Verhalten des analogen Teils der Elektronik kann bei Bedarf auch durch Baugruppen verbessert werden, die eine Pol-Nullstellen-Kompensation direkt vornehmen. Dadurch kann Rauschen reduziert werden, ein ungünstiges Übertragungsverhalten kann verbessert werden oder können nach bestimmten Kriterien optimale Übertragungseigenschaften erreicht werden.

## 3. Hochpaßfilter

Die spektrale Begrenzung des Eingangssignals bezogen auf die tiefen Frequenzen ist erfindungsgemäß aus drei Gründen günstig:

1. 1/f-Rauschen

Die Amplitude des 1/f-Rauschens wächst reziprok zur Frequenz an. Daher treten mit zunehmender Meßzeit Rauschanteile mit immer tieferer Frequenz auf und verfälschen das zu messende Signal. Die Hauptquellen für das 1/f-Rauschen sind der Sende-Oszillator, der Konverter-Oszillator, die Operationsverstärker.

2. Langsame Bewegungen

Bewegungen des zu detektierenden Körpers führen bei konstanter Geschwindigkeit zu einer Doppler-Frequenzverschiebung und damit zu Spektralkomponenten, die in das zu untersuchende Frequenzband fallen können. Bei unregelmäßigen Bewegungen tritt ein breites Zusatzband auf. Je langsamer die Bewegungen, desto tieffrequenter die Spektren, die dann immer schwerer von Rauschanteilen zu trennen sind.

3. Auswertezeit

Um eine Spektrallinie der Frequenz f zu identifizieren, muß mindestens eine Zeit t=1/f gemessen werden, d.h. je tiefer die nachzuweisenden Frequenzen, desto länger muß gemessen werden. Da nicht garantiert werden kann, daß die Meßzeit ein ganzzahliges Vielfaches der interessierenden Spektralkomponente ist, tritt ein Leckeffekt bei der Fourier-Analyse auf. Dieser führt zu einer spektralen Spreizung. Daher muß bei der Analyse tiefer Frequenzen eine Meßzeit eingehalten werden, die ein Vielfaches der Periodendauer beträgt, wobei die Genauigkeit mit der Meßzeit proportional zunimmt. Bei 10% Fehler in der spektralen Auflösung und 0,2 Hz unterer Frequenz muß mit typischen 50 Sekunden Meßzeit gerechnet werden.

Den allgemeinen Aufbau der Auswertekette zeigt die Figur 3. Als Zentraleinheiten wurden Personalcomputer aus dem Bürobereich, Typ.IBM-PC-Kompatible, eingesetzt, da deren Leistungsfähigkeit für die gestellte Aufgabe ausreichte.

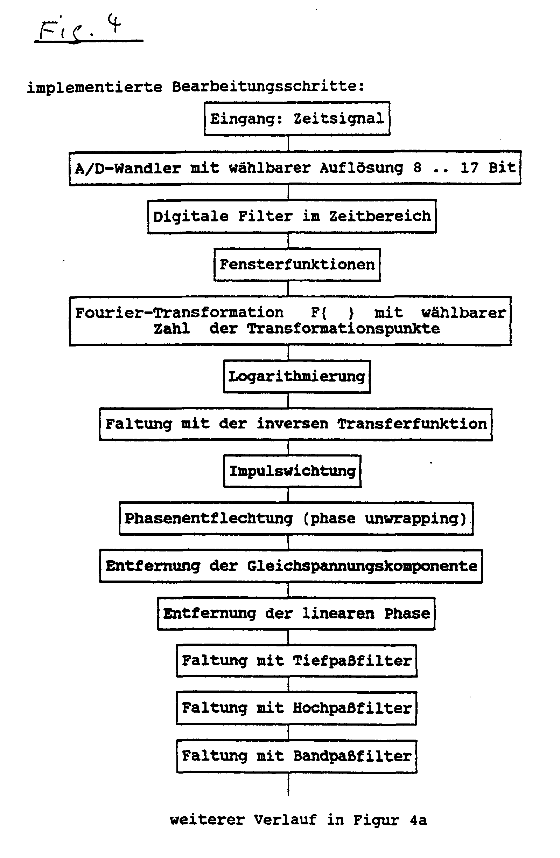

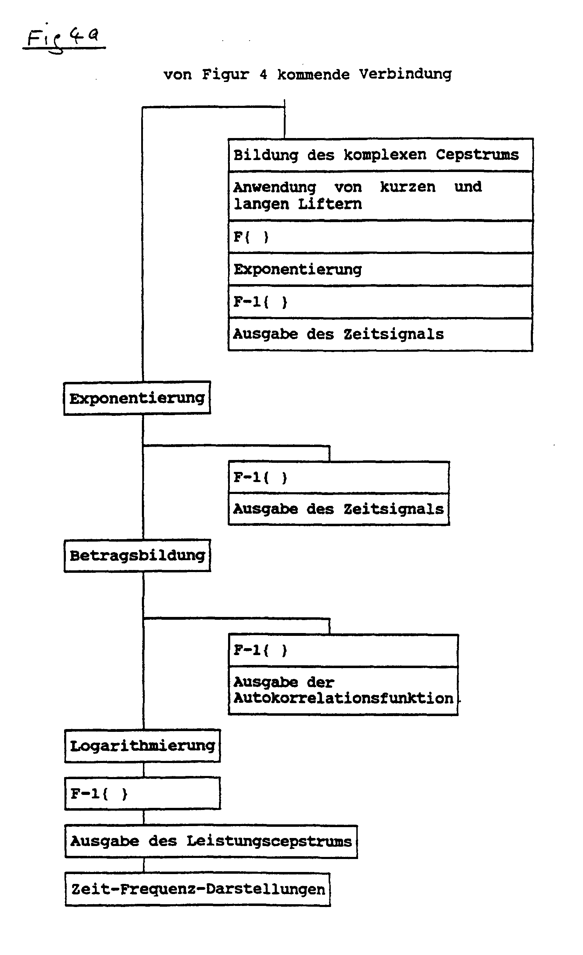

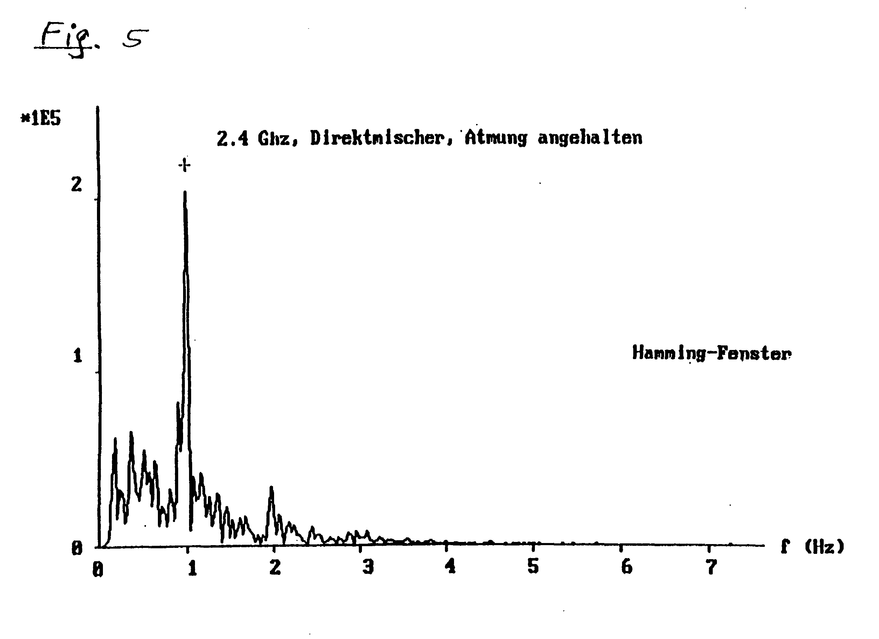

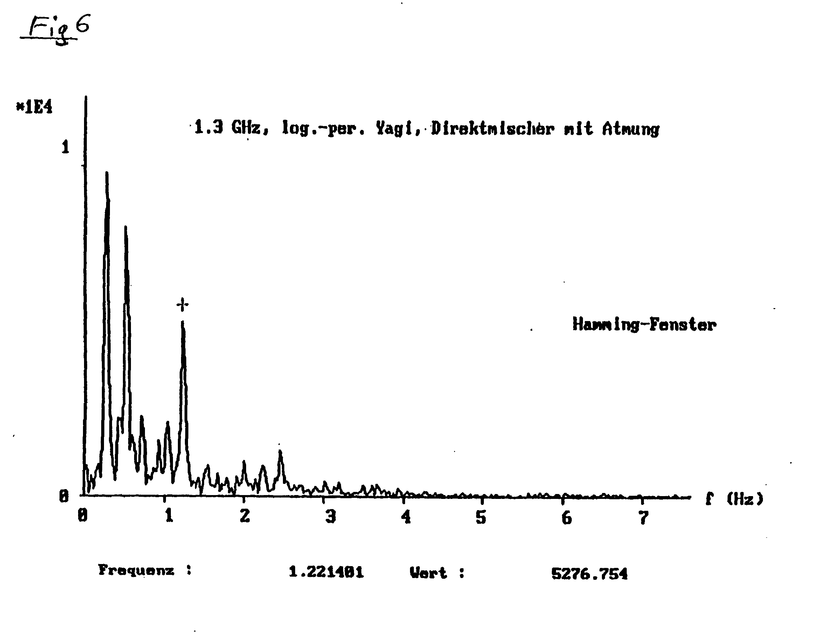

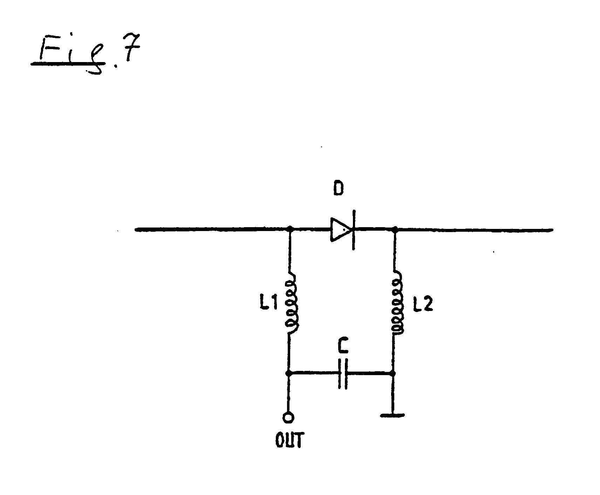

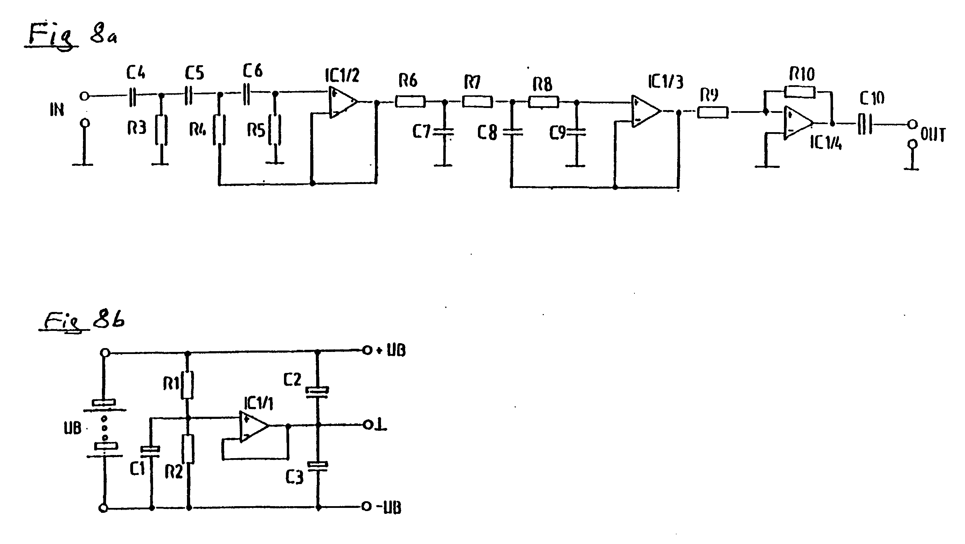

Der in Figur 4 und 4a dargestellte Plan gibt eine Übersicht über die implementierten Bearbeitungsschritte, hierbei bezeichnet F { } die Fourier-Transformation und F-1 { } die inverse Fourier-Transformation.

Ergebnisse

Nach verschiedenen Vorversuchen stellte sich eine Abtastrate von 16 Hz bei einer unipolaren Auflösung von 13 Bit (Gesamtauflösung 14 Bit) als gut geeignet heraus. Als Fensterbreite für die Spektralanalyse wurden 512 Werte entsprechend etwa 33 Sekunden gewählt; als Fenster das Hamming-Fenster.

Figur 5 zeigt die Herzfrequenz einer Versuchsperson bei angehaltener Atmung. Die spektrale Komponente hebt sich so deutlich von der Umgebung ab, daß für den Nachweis des Herzschlags der Versuchsperson eine weitere Bearbeitung nicht notwendig ist. Aufgetragen ist das Betragsspektrum in willkürlichen Einheiten über der Frequenz in Hertz. Die Messung erfolgte bei 2,4 GHz, es wurde der Diodendirektempfänger, d.h. der 1/2-Dipol als Empfänger benutzt, als Sender der lokaler Oszillator, die Atmung wurde angehalten.

Figur 6 zeigt das Spektrum des von einer atmenden Person reflektierten Signals unter Einsatz des Diodendirektempfängers und der logarithmisch-periodischen Yagi-Antenne sowie des 1,3 GHz Sendeozillators als Quelle. Sowohl Herzfrequenz als auch Atmungsfrequenz sind vorhanden.

Bei der Frequenz 440 MHz gestalteten sich die Versuche aufgrund der extremen Empfindlichkeit der gesamten Anordnung als schwierig. Fast alle Versuchsaufnahmen zeigen Übersteuerungen und Reaktionen auf externe Ereignisse.

Das Problem der Übersteuerung ist durch entsprechende Dämpfung lösbar; der Nachweis der Atmungs- und Herztätigkeit wird dadurch nicht beeinflußt.

Wird ein Zirkulator eingesetzt, so kommt man wie beschrieben mit einer Antenne, die gleichzeitig sendet und empfängt, aus.

Die Beispiele belegen anschaulich, daß die Detektion lebender Personen möglich ist. Dabei sind weder Mauern noch Entfernungen von einigen 10 Metern ein nennenswertes Hindernis. Als Arbeitsfrequenz stellten sich 1,3 GHz und 2,4 GHz als gut geeignet heraus. Bei noch handlichen Antennen ist die Empfindlichkeit hoch genug, um reproduzierbare Ergebnisse mit eindeutiger Identifikation des Herzschlags und der Atmung zu erhalten, ohne daß intensive numerische Bearbeitungsschritte notwendig sind, da bereits entsprechend starke Empfangssignale vorliegen.

Schaltplan Hochpaß und Anti-Alisaing-Tiefpaß

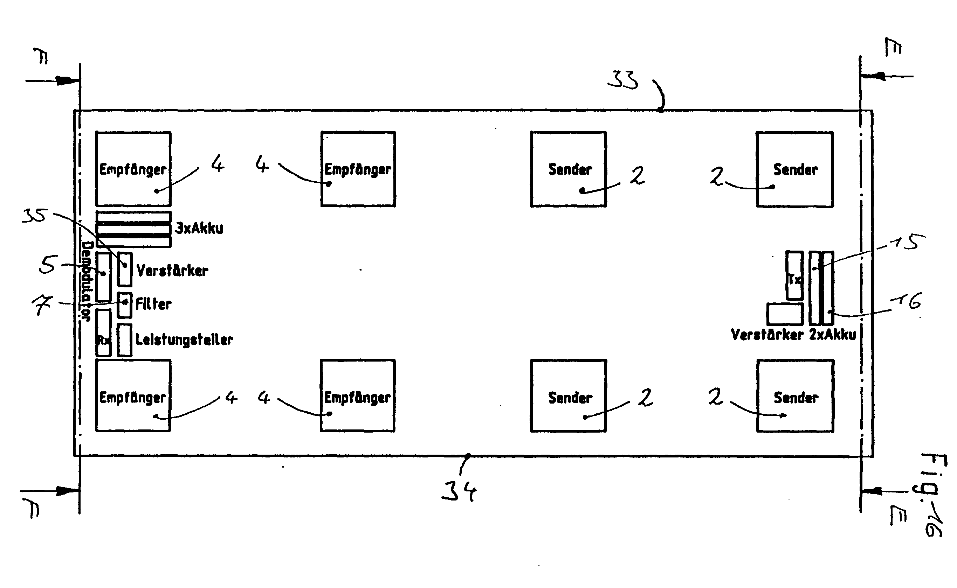

Der Schaltplan der zur Bandbegrenzung eingesetzten Baugruppe ist in Fig. 8a und 8b wiedergegeben. Der Hochpaß dritter Ordnung unterdrückt die tieffrequenten Rauschanteile, insbesondere des 1/f-Rauschens. Der folgende Tiefpaß dritter Ordnung begrenzt das Spektrum zu höheren Frequenzen. Es folgt eine lineare Verstärkerstufe zur Pegelangleichung. Die Betriebsspannung wird elektronisch symmetriert, so daß eine unipolare Versorgung ausreicht. Zwei dieser Baugruppen in Kaskade erfüllen die Anforderungen, die das Abtasttheorem stellt.

Schaltplan Diodendemodulator

Zur Phasendemodulation des auf die Zwischenfrequenz gemischten Empfangssignals und als Direktdemodulator für die entwickelten Empfangsantennen dient ein Diodendetektor, dessen Schaltung in Figur 7 zu sehen ist. Die Schaltung entspricht einem typischen Leistungsmesser; ein Vorstrom kann vom Ausgang eingeprägt werden. Die Eingangsimpedanz kann an den ZF-Mischer oder die Antennen angepaßt werden.

Schaltplan Diodendirektempfänger

Die Diodendirektempfänger bestehen aus 1/2 oder 1 Wellenlängen langen, mit dem entsprechenden Verkürzungsfaktor multiplizierten Diodendetektoren und sind entsprechend vorgeschaltet. Am Ausgang kann ein Vorstrom eingeprägt werden.

Außerdem wurde jede Einheit mit einer eigenen stabilisierten Spannungsversorgung sowie einem eigenen Ein-Aus-Schalter versehen, so daß Baugruppen mit großer Zeitkonstante (Lokale Oszillatoren, Vorverstärker, Tiefpaß) im Dauerbetrieb gefahren werden konnten und sich im thermischen und elektrischen Gleichgewicht befanden, während Verbraucher mit hoher Stromaufnahme (Sendeendstufen, Konverter) zwischen den Anwendungen abgeschaltet werden können.

Nachfolgend werden bevorzugte Ausführungsweisen der Erfindung beschrieben.

Eine erste erfindungsgemäße Ausführungsweise umfaßt die Verwendung eines Systems zur Überwachung der Vitalfunktion von Atmung und/oder Herzschlag im medizinischen Bereich. In einem Gehäuse 14 befinden sich die Sendeantenne 2 sowie die Empfangsantenne 4 mit ihren jeweiligen zugehörigen Reflektoren. Die als Sendeantenne verwendete Antenne 2 ist mit einem Sender 1 verbunden, der an einer Ersatzlast von reellen 377 Ω eine Leistung von 20 mW abgibt.

Der horizontale und vertikale Öffnungswinkel der Antennen sind der jeweiligen Anwendung angepaßt. Zur Überwachung auf Intensivstationen weist zumindest die Empfangsantenne eine Empfangscharakteristik auf, die geringe Nebenkeulen hat und deren Haupterfassungsgebiet in etwa die Größe eines menschlichen Thorax hat. Die Empfangsantenne 4, Rx ist mit einem Empfänger verbunden, der mittels des Konverters 6 die eingehenden Signale in den vorstehend beschriebenen Frequenzbereich konvertiert. Daran schließen sich der Demodulator 5, der Verstärker, der Filter 7 und ein Treiber für die Signalübertragung an.

Mittels in den Figuren nicht dargestellten, abgeschirmten Zuleitungen werden die Signale dem entfernt angeordneten Analog/Digital-Wandler 9 zugeführt. Der Analog/Digital-Wandler 9 sowie die weitere vorstehend beschriebene Auswertungselektronik sind entweder in einer portablen Einheit, vorzugsweise in einem Handkoffer oder in einem stationären netzgespeisten Gerät untergebracht, oder sind Teil einer zentralen Überwachungseinheit mit zentraler Anzeige der Vitalfunktionen in Form der in Fig. 5 und 6 dargestellten Spektren in einer klinischen Überwachungsstation. Um die Signale im Zeitbereich korrekt auswertbar zu machen wird eine Faltung mit der inversen Transferfunktion des vor der Analog/Digitalwandlung liegenden Signalpfades durchgeführt. Somit kann auf Monitoren der zentralen Überwachungsstaion oder des portablen Gerätes der im wesentlichen momentane Zustand der Vitalfunktionen oder mit entsprechenden Bildspeichereinrichtungen deren Historie dargestellt werden.

Die Frontplatte 15 trägt in einer einfacheren Ausführungsform wie in Fig. 10 dargestellt einen Ein/Aus-Schalter 16 sowie einen optischen Indikator 17 und/oder einen akustischen Indikator 18. Der optische Indikator 17 kann entweder, wie in Fig. 10 gezeigt, unter einem transparenten Teil der Frontblende 15 oder oberhalb des Gehäuses 15, wie in Fig. 9 dargestellt, angeordnet sein. Mittels eines Klemmanschlusses 19, der in Fig. 9 lediglich symbolisch als Schraubklemmvorrichtung dargestellt ist, kann diese erste erfindungsgemäße Ausführungsform zügig und auf einfache Weise an oder in der Nähe von Betten von zu überwachenden Patienten befestigt werden. Es liegt darüber hinaus im Rahmen der Erfindung, für die Befestigungsvorrichtung 19 beliebige andere bekannte Alternativen, wie beispielsweise mechanische Kupplungen oder Bajonette mit dem jeweiligen ortsfest montierten Gegenstück, einzusetzen. Derart kann auch im heimischen Bereich die Vorrichtung an oder in der Nähe des Bettes der zu überwachenden Person befestigt werden.

Auf an sich bekannte Weise gestattet ein Gelenkarm 21 zusammen mit dem Drehkippgelenk 22 am Ende des Arms 21 das gezielte Ausrichten der Vorrichtung.

In einer weiteren, in Fig. 11 dargestellten Ausführungsform ist das Gehäuse 15 in etwa mittig mit einer Zugvorrichtung 23 an einer Gebäudedecke 24 befestigt. Die Zugvorrichtung 23 gestattet die Höheneinstellung, insbesondere das Herunterfahren des Gehäuses 15, so daß eine unter der Vorrichtung liegende Person optimal erfaßbar ist, bei nach oben verschobener Vorrichtung jedoch keine Behinderung der Bewegungsfreiheit eintritt.

Um den klinischen Gegebenheiten sicher Rechnung tragen zu können, wird die erfaßte Bandbreite der Frequenzen auf einen Bereich von 0,02 Hz bis 6 Hz beschränkt. Hierdurch sind Atmungsperioden von 166 ms bis 50 s und Pulsraten von 1,2 bis 360 Schläge pro Minute detektierbar. Eine nachgeschaltete Auswertungselektronik erfaßt die Höhe der spektral beschränkten Signale und verfügt über eine

Schwellenwerteinstellung, die beim Ausbleiben der die Vitalfunktionen warnenden Signale, einen optischen und/oder akustischen Alarm veranlaßt oder bei Integration in ein Überwachungssystem den Alarmzustand an dieses weiterleitet.

In weiterer alternativer Ausgestaltung werden die erfaßbaren Atmungsperioden auf weniger als 30 s beschränkt, so daß auch der Zeitraum bis zur Auswertung der Signale nicht länger als etwas über 30 s beträgt.

Die Haupteinsatzgebiete der vorstehend beschriebenen Verfahren betreffen die Überwachung von suizidgefährdeten Personen, die kabelfreie Überwachung von komatösen Personen sowie von Personen mit Verbrennungen. Im klinischen sowie heimischen Bereich kann die Überwachung von schwer und schwerst Pflegebedürftigen erfolgen sowie bei Kleinkindern eine Überwachung in bezug auf das Ausbleiben der Vitalfunktion zur Vermeidung des plötzlichen Kindstodes. Von

Intensivstationen bekannte, durch die Verkabelungen hervorgerufene Schlafstörungen können gemildert und zum Teil sogar vermieden werden, da neben der rein mechanischen Behinderung auch die psychische Belastung abnimmt.

Auch die Erfassung der Herzfrequenz des ungeborenen ist bei Schwangerschaftsuntersuchungen möglich. Hierdurch wird auch eine beschwerdefreie sowie lückenlose Dauerüberwachung ohne die herkömmlich anzulegenden Elektroden ermöglicht.

Bei einer weiteren in Fig. 13 dargestellten Ausführungsform wird ein Raum 25 mittels einer in einer Deckenecke angeordneten Sende/Empfangseinrichtung überwacht. Wie bei den vorhergehenden Ausführungsformen beschrieben, ist die Sendeantenne 2 sowie die Empfangsantenne 4 mit den vorstehend elektronischen Baugruppen zur Signalauswertung verbunden.

Der Sender 1 sendet vorzugweise im Frequenzbereich von 300 MHz bis 3 GHz, dieser gibt an einer Ersatzlast von reellen 50 Ω oder reellen 377 Ω eine Leistung von einigen mW bis mehreren W ab. Im Falle der Raumüberwachung sind die Antennen 2, 4 als Tripol-Prisma, wie in Fig. 13 dargestellt, ausgebildet.

In der in Fig. 14 dargestellten weiteren Ausführungsform ist im Dachgeschoß 26 des Gebäudes 27 eine zirkularpolarisierende Sende/Empfangsantenne 2, 4 angeordnet. Durch die entsprechende Ausbildung der räumlichen Sendecharakteristik der Sendeantenne 2 sowie der räumlichen Empfangscharakteristik der Antenne 4 werden diese an das erwünschte Erfassungsgebiet, in der Regel die Abmessungen des zu überwachenden Gebäudes, angepaßt. Es können darüber hinaus mehrere Sendeantennen 2, sowie mehrere Empfangsantennen 4 in den zu überwachenden Bereichen angeordnet und jeweils mit der Auswertungselektronik verbunden sein.

In der in Fig. 15 dargestellten Ausführungsform sind hinter einer abgehängten Decke 28 die Sende- oder die Empfangsantenne 2, 4 angeordnet. Durch deren großflächige Erstreckung wird eine genaue Definition des Erfassungsgebietes vereinfacht. In Fig. 15 ist ebenfalls im Bereich der Seitenwand 29 eine weitere verdeckte Überwachungsanordnung gezeigt.

In Fig. 16 ist zusätzlich zur Überwachung des Raumes 30 eine Sende-Empfangsantennenkombination 2, 4 für die Vorfeldüberwachung gezeigt. Der nur schematisch dargestellte Bereich 30 kann hierbei ein Vorplatz eines privaten sowie öffentlichen Gebäudes sein. So ist beispielsweise im Strafvollzug oder in der Psychiatrie eine flächendeckende ununterbrochene Überwachung möglich. Darüber hinaus kann eine nachgeschaltete Auswertungselektronik, wie vorstehend für die medizinischen Anwendungsbereiche geschildert, selbsttätige Signalisierung bei durchführen.

Weitere wichtige Anwendungsgebiete liegen im Bereich der chemischen Industrie sowie bei strahlungsexponierten Anlagen der nuklearen Energieversorger oder der Nuklearbrennstoffverund -aufbereiter. Chemisch- oder strahlungsexponierte Bereiche können flächendeckend überwacht werden, so daß diese Bereiche nur bei entsprechender Anmeldung ohne Auslösung eines Alarms betreten werden können oder beim Austreten gefährlicher Stoffe in der Nähe der Austrittsstelle das Vorhandensein lebender Menschen erfassbar ist.

Eine weitere wesentliche Anwendung liegt im Bereich der gezielten Brandbekämpfung. Überall dort wo zur Vermeidung größerer Sachschäden anstelle konventioneller, sauerstoffbindende Löschmittel oder spezielle Löschverfahren zur Brandbekämpfung eingesetzt werden (z.B. im Falle eines Brandes in der Druck- und Computerindustrie), darf, um das Leben noch in den Gebäuden befindlicher Personen nicht zu gefährden, der Löscheinsatz erst beginnen, wenn eine Überprüfung der Brandlokalitäten auf das Vorhandensein lebender Personen durchgeführt wurde. Diese Kontrolle ist für die Verwendung spezieller Löschmittel gesetzlich vorgeschrieben, aber aufgrund der damit verbundenen Nachteile für Leib und Leben der Feuerwehreinsatzleute stark umstritten. Eine Überprüfung der Brandlokalitäten auf noch lebende Personen kann hingegen effizient, schnell und von außen, d.h. auch von außerhalb der brennenden Räumlichkeiten, durchgeführt werden. Somit werden in den brennenden Räumlichkeiten befindliche Personen schneller erkannt, zügiger gerettet und die Feuerwehreinsatzleute am Brandherd weniger gefährdet. Durch den schnelleren Löschbeginn kann zusätzlich in der Regel das Ausmaß des Brandschadens minimiert werden.

**Patentansprüche**

1. Verfahren zur Erfassung von Vitalfunktionen lebender Körper, insbesondere zur Erfassung von Vitalfunktionen lebender menschlicher Körper, mit Hilfe des Empfangs elektromagnetischer Signale, wobei ein empfangenes elektromagnetisches Signal an einem Bauteil mit nichtlinearer Kennlinie direkt, ohne ein zusätzliches externes Referenzsignal demoduliert wird, und für lebende Körper charakteristische Frequenzanteile in einem Frequenzbereich von etwa 0,01 Hz bis etwa 10 Hz, vorzugsweise von etwa 0,2 Hz bis etwa 3 Hz, aus dem demodulierten, empfangenen, elektromagnetischen Signal gewonnen werden.

2. Verfahren nach Anspruch 1, **dadurch gekennzeichnet, daß** das empfangene elektromagnetische Signal auf eine Zwischenfrequenz umgesetzt wird.

3. Verfahren nach einem der Ansprüche 1 bis 2, **dadurch gekennzeichnet, daß** das empfangene Signal analog zu hohen und zu niedrigen Frequenzen hin begrenzt wird.

4. Verfahren nach Anspruch 3, **dadurch gekennzeichnet, daß** das empfangene elektromagnetische Signal nach der Filterung abgetastet und in ein digitales Signal gewandelt wird.

5. Verfahren nach Anspruch 4, **dadurch gekennzeichnet, daß** das digitale Signal mit einer Fensterfunktion im Zeitbereich, und mit der inversen Transferfunktion der Empfangseinrichtung gefaltet wird.

6. Verfahren nach Anspruch 3, 4 oder 5, **dadurch gekennzeichnet, daß** das digitale Signal vor dessen Auswertung und Darstellung als Ausgangssignal vom Zeitin den Frequenzbereich transformiert wird.

7. Verwendung eines Verfahrens nach den Ansprüchen 1 bis 6 in der Intensivmedizin zur berührungsfreien Überwachung der Vitalfunktionen von Intensivpatienten.

8. Verwendung eines Verfahrens nach den Ansprüchen 1 bis 6 zur Überwachung von Kleinkindern oder Apnoe-Patienten auf Atem- und/oder Herzstillstand.

9. Verwendung eines Verfahrens nach den Ansprüchen 1 bis 6 im Justizvollzug zur Überwachung des Zustands Inhaftierter.

10. Verwendung eines Verfahrens nach den Ansprüchen 1 bis 6 zur Überwachung und/oder Sicherung von Räumen, Gebäuden und/oder diese umgebendem Freiland.

**Claims**

1. A method for detecting vital functions of living bodies, in particular for detecting vital functions of living human bodies, by means of receiving electromagnetic signals, and

by directly demodulating a received electromagnetic signal by a component having a non-linear current/voltage characteristic without an additional external reference signal, and

by obtaining frequency components which are characteristic of the living bodies from the demodulated received electromagnetic signal within a frequency range of from about 0.01 Hz through about 10 Hz, preferably from about 0.2 Hz through about 3 Hz.

2. The method as set forth in claim 1 further **characterised by** converting the received electromagnetic signal to an intermediate frequency.

3. The method as set forth in claim 1 or 2 further **characterised by** limiting the received electromagnetic signal with an analogue device towards high and low frequencies.

4. The method as set forth in claim 3 further **characterised by** sampling and converting the received, filtered electromagnetic signal into a digital signal.

5. The method according to claim 4 further **characterised by** folding the digital signal with a window function in a time region and with an inverse transfer function of a receiving device.

6. The method as set forth in claim 3, 4 or 5 further **characterised by** transforming the digital signal from a time region into a frequency region and then evaluating and representing the digital signal as output signal.

7. Use of a method as set forth in any of the claims 1 to 6 for contact-free monitoring of the vital functions of intensive care patients in the intensive care.

8. Use of a method as set forth in any of the claims 1 to 6 for monitoring at least one of small children and apnoea patients for at least one of cardiac and respiratory arrest.

9. Use of a method as set forth in any of the claims 1 to 6 for monitoring the state of inmates in places of detention.

10. Use of a method as set forth in any of the claims 1 to 6 for at least one of monitoring and safeguarding at least one of rooms, buildings and open land surrounding same.

**Revendications**

1. Procédé pour l'acquisition de fonctions vitales de corps vivants, notamment pour l'acquisition de fonctions vitales de corps humains vivants, à l'aide de la réception de signaux électromagnétiques, dans lequel un signal électromagnétique reçu est démodulé directement sur un composant à courbe caractéristique non linéaire, sans signal de référence externe supplémentaire, et grâce auquel sont acquis des composants harmoniques caractéristiques des corps vivants dans une plage de fréquences d'environ 0,01 Hz à environ 10 Hz, de préférence entre environ 0,2 Hz et environ 3 Hz, extraits du signal électromagnétique reçu et démodulé.

2. Procédé selon la revendication 1, **caractérisé par le fait que** le signal électromagnétique reçu est transformé vers une fréquence intermédiaire.

3. Procédé selon les revendications 1 à 2, **caractérisé par le fait que** le signal reçu est limité de manière analogique entre des fréquences hautes et des fréquences basses.

4. Procédé selon la revendication 3, **caractérisé par le fait que** le signal électromagnétique reçu est échantillonné après le filtrage et converti en signal numérique.

5. Procédé selon la revendication 4, **caractérisé par le fait que** l'on fait le produit de convolution du signal numérique avec une fonction de fenêtrage temporel et avec la fonction de transfert inverse du dispositif de réception.

6. Procédé selon les revendications 3, 4 ou 5, **caractérisé par le fait que** le signal numérique est transformé du domaine temporel dans le domaine de fréquences avant son analyse et sa représentation en tant que signal de sortie.

7. Utilisation d'un procédé selon les revendications 1 à 6 en médecine de soins intensifs pour la surveillance sans contact des fonctions vitales de patients de soins intensifs.

8. Utilisation d'un procédé selon les revendications 1 à 6 pour la surveillance d'arrêts respiratoires et/ou cardiaques chez des enfants en bas âge ou des patients souffrant d'apnées.

9. Utilisation d'un procédé selon les revendications 1 à 6 dans des établissements pénitentiaires pour la surveillance de l'état de détenus.

10. Utilisation d'un procédé selon les revendications 1 à 6 pour la surveillance et/ou la sécurisation de locaux, bâtiments et/ou de leur environnement extérieur.

**Fig. 1**

**Fig. 2**

*Fig 3*

**Aufbau der Auswertekette**

```
        ┌─────────────────────────┐
        │   Analoger Eingang       │
        └────────────┬────────────┘
                     │
        ┌────────────┴────────────┐
        │   Tiefpaß-Filter         │
        │   Anti-Aliasing          │
        └────────────┬────────────┘
                     │
        ┌────────────┴────────────┐
        │   Abtast- und            │
        │   Haltebaustein          │
        ├─────────────────────────┤
        │   Analog/Digital         │
        │   Wandler                │
        └────────────┬────────────┘
                     │
        ┌────────────┴────────────┐
        │   Digitale Signal-       │
        │   verarbeitung           │
        └────────────┬────────────┘
                     │
        ┌────────────┴────────────┐
        │   Auswertung             │
        └────────────┬────────────┘
                     │
        ┌────────────┴────────────┐
        │   Digitaler Ausgang      │
        └─────────────────────────┘
```

*FIG. 4*

implementierte Bearbeitungsschritte:

| Eingang: Zeitsignal |

| A/D-Wandler mit wählbarer Auflösung 8 .. 17 Bit |

| Digitale Filter im Zeitbereich |

| Fensterfunktionen |

| Fourier-Transformation F( ) mit wählbarer Zahl der Transformationspunkte |

| Logarithmierung |

| Faltung mit der inversen Transferfunktion |

| Impulswichtung |

| Phasenentflechtung (phase unwrapping) |

| Entfernung der Gleichspannungskomponente |

| Entfernung der linearen Phase |

| Faltung mit Tiefpaßfilter |

| Faltung mit Hochpaßfilter |

| Faltung mit Bandpaßfilter |

weiterer Verlauf in Figur 4a

Fig 4a

von Figur 4 kommende Verbindung

| |
|---|
| Bildung des komplexen Cepstrums |
| Anwendung von kurzen und langen Liftern |
| F( ) |
| Exponentierung |
| F-1( ) |
| Ausgabe des Zeitsignals |

Exponentierung

| |
|---|
| F-1( ) |
| Ausgabe des Zeitsignals |

Betragsbildung

| |
|---|
| F-1( ) |
| Ausgabe der Autokorrelationsfunktion |

Logarithmierung

F-1( )

Ausgabe des Leistungscepstrums

Zeit-Frequenz-Darstellungen

Fig. 5

2.4 Ghz, Direktmischer, Atmung angehalten

Hamming-Fenster

EP 0 740 800 B1

Fig 6

*1E4

1

1.3 GHz, log.-per. Yagi, Direktmischer mit Atmung

Hamming-Fenster

0

0    1    2    3    4    5    6    7         f (Hz)

Frequenz :         1.221481     Wert :         5276.754

Fig.7

EP 0 740 800 B1

Fig. 8a

Fig. 8b

Fig. 9

Fig. 12, 13

Fig. 11

Fig. 14

Fig. 10

Fig. 15

Fig. 17

Fig. 16

Fig.11

EP 0 740 800 B1

Fig. 12

Fig. 13

Demodulator

Akku

Akku

Verstärker

Treiber

Akku

Akku

Filter

Fig. 14

Antenne Rx

Rx

Tx

Akku | Akku | Demo-dulator | Filter | Ver-stärker

Fig. 15

EP 0 740 800 B1

Fig. 16

27

Fig. 17

3 3

①

34

Antenne

4

3xAkku

Demodulator

Rx

5

Antenne

4

①

EP 0 740 800 B1